# EUROPEAN PATENT SPECIFICATION

(11) **EP 2 972 324 B1**
(45) Date of publication and mention of the grant of the patent: **22.04.2020**
(21) Application number: 14717367.8
(22) Date of filing: 14.03.2014
(51) Int. Cl.: G01N 33/50, C12Q 1/28

(54) **A NONINVASIVE METHOD FOR DIAGNOSING A DANDRUFF CONDITION**
NICHT-INVASIVES VERFAHREN ZUR DIAGNOSE VON KOPFSCHUPPEN
PROCÉDÉ NON INVASIF POUR LE DIAGNOSTIC DES PELLICULES

(30) Priority: 15.03.2013 US 201361793889 P
(43) Date of publication of application: 20.01.2016
(60) Divisional application: 17196627.8
(73) Proprietor: The Procter & Gamble Company, Cincinnati, OH 45202 (US)
(72) Inventor: KERR, Kathleen, Marie, Cincinnati Ohio 45202 (US); FIENO, Angela, Marie, Cincinnati Ohio 45202 (US); WEHMEYER, Kenneth, Robert, Cincinnati Ohio 45202 (US); LI, Lijuan, Cincinnati Ohio 45202 (US); SCHWARTZ, James, Robert, Cincinnati Ohio 45202 (US); GRANT, Raymond, Alan, Cincinnati Ohio 45202 (US)
(74) Representative: P&G Patent Germany
(86) International application number: PCT/US2014/028598
(87) International publication number: WO 2014/144265

(56) References cited:
- WO-A1-2004/041231
- US-A1- 2010 267 825
- US-B1- 6 262 041
- TRUSH M A ET AL: "Myeloperoxidase as a biomarker of skin irritation and inflammation", FOOD AND CHEMICAL TOXICOLOGY, PERGAMON, GB, vol. 32, no. 2, 1 February 1994 (1994-02-01), pages 143-147, XP025532043, ISSN: 0278-6915, DOI: 10.1016/0278-6915(94)90175-9 [retrieved on 1994-02-01]
- JANG M ET AL: "EFFECTS OF RESVERATROL ON 12-O-TETRADECANOYLPHORBOL-13-ACETATE- INDUCED OXIDATIVE EVENTS AND GENE EXPRESSION IN MOUSE SKIN", CANCER LETTERS, NEW YORK, NY, US, vol. 134, no. 1, 11 December 1998 (1998-12-11), pages 81-89, XP000856519, ISSN: 0304-3835, DOI: 10.1016/S0304-3835(98)00250-X
- PERIHAN OZTURK ET AL: "Local oxidative stress in interdigital tinea pedis", THE JOURNAL OF DERMATOLOGY, vol. 40, no. 2, 1 February 2013 (2013-02-01), pages 114-117, XP055125998, ISSN: 0385-2407, DOI: 10.1111/1346-8138.12043
- KATHY KERR ET AL: "Epidermal changes associated with symptomatic resolution of dandruff: biomarkers of scalp health", INTERNATIONAL JOURNAL OF DERMATOLOGY, vol. 50, no. 1, 23 December 2010 (2010-12-23), pages 102-113, XP055031486, ISSN: 0011-9059, DOI: 10.1111/j.1365-4632.2010.04629.x
- JIN-OK BAEK ET AL: "Assessment of an imiquimod-induced psoriatic mouse model in relation to oxidative stress", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 304, no. 9, 5 August 2012 (2012-08-05), pages 699-706, XP035130141, ISSN: 1432-069X, DOI: 10.1007/S00403-012-1272-Y
- Perihan Ozturk ET AL: "ACTA DERMATOVENEROLOGICA CROATICA ACTA DERMATOVENEROLOGICA CROATICA Oxidative Stress in Patients with Scalp Seborrheic Dermatitis", Acta Dermatovenerol Croat, vol. 21, no. 2, 17 February 2013 (2013-02-17), pages 80-85, XP055355386,

## Description

### FIELD OF THE INVENTION

The present invention relates to a method for diagnosing dandruff condition by measuring the amount of the biomarker Myeloperoxidase, associated with oxidative stress and/or oxidative damage from a skin sample and the link and correlation of the amount of this biomarker as it directly correlates to improvement in dandruff condition in mammals.

### BACKGROUND OF THE INVENTION

The scalp/skin is a remarkable organ system composed of multiple specialized tissues that function as a first line of defense against environmental insults. While early research focused primarily on the barrier function of the skin, it has become clear that that this organ is dynamic: sensing and responding to even small changes in the environment in order to help maintain homeostasis. Environmental influences such as solar radiation, pollution, or even the application of skin care products, result in a complex cascade of events that ultimately lead to changes in the expression of hundreds or thousands of genes. These changes in gene expression are generally translated to changes in protein production (or accumulation, release, modification etc.) that catalyze chemical reactions ultimately leading to the cellular response. As these chemical reactions proceed, metabolic byproducts are often left as an indicator of what chemical processes have taken place. Analysis of these resulting proteins and small molecule biomarkers which correlate to a given skin condition, for example, dandruff can provide valuable information in understanding the condition as well as developing products for the purpose of diagnose and/or improve the skin condition. Dandruff is a common chronic relapsing scalp skin condition with flaking and itching sensations. The pathogenesis of dandruff is complex, and appears to be the result of interactions among scalp skin, microflora and the host immune system. Much of the previous work on this condition has focused on the examination of a few surface-level phenomena. Traditional expert- and self-observation-based assessments are combined with largely instrumental-based assessments of epidermal structure and function at the physiological level. New biomolecular capabilities establish a depth of pathophysiological understanding not previously achievable with traditional means of investigation; however, a clear picture of the molecular events leading to the key symptoms of this condition has yet to emerge. To elucidate these key molecular events bimolecular sampling can be obtained noninvasively by tape stripping of the skin surface followed by chemical or bioanalytical methodologies. Histamine was recently identified as a sensitive biomarker for scalp itch. Additional biomarkers are needed to enable a more detailed pathophysiological description of the dandruff condition as well as serve as relevant measures indicative of the extent and completeness of therapeutic resolution of dandruff. One such area of interest is oxidative stress. It is well known in the literature that oxidative stress is damaging to skin and that antioxidants can provide a protective effect from various sources of oxidative damage. The formation of free radicals is a widely accepted mechanism leading to skin aging and damage. Free radicals are highly reactive molecules with unpaired electrons that can directly damage various cellular structural membranes, lipids, proteins, and DNA. The damaging effects of these reactive oxygen species are induced internally during normal metabolism and immune response and externally through various oxidative stressors like sun exposure, first- or secondhand cigarette smoke, environmental toxins, poor diet, stress, etc. These various reactive oxygen species must be continually removed from cells to maintain healthy metabolic function. The body has several ways to deal with this 1) to remove the reactive oxygen species, 2) sequester iron and other metals, 3) to scavenge formed radicals, and 4) to repair molecular damage. Oxidative stress results when the balance between production of reactive oxygen species and antioxidant defenses against them is disturbed. Free radicals also lead to inflammation, which is considered to play an additional role in skin aging and is detrimental to general skin health. The production of free radicals increases with age, while the integrity and ability of endogenous defense mechanisms to counter them decreases. An imbalance leads to progressive and cumulative damage to cellular structures resulting in aging and accelerated aging where environmental aging is superimposed on the natural aging process.

Oxidative stress and resulting molecules that are generated as a result of oxidative damage (e.g., oxidized lipids) can be assessed by biomarkers which are sampled noninvasively, enabling their use in routine clinical evaluations.

In order to assess oxidative stress for the dandruff condition, an antibody-based assay was used to quantify the protein myeloperoxide (MPO). MPO is a pro-inflammatory enzyme that is secreted by neutrophils (PMNs) as part of the host defense mechanism against a wide range of microbial pathogens (e.g., *Malassezia*). MPO can transform hydrogen peroxide into the bactericidal agent, hypochlorous acid, plus an array of potentially damaging reactive oxygen species (ROS). In addition, MPO may function as an antimicrobial peptide or protein. Although the mechanism is not completely understood, it is reasonable to hypothesize that the reactive oxygen species generated by MPO may cause collateral damage to the surrounding tissue while killing pathogens. In order to assess the oxidative damage resulting from oxidative stress an High Performance Liquid Chromatography with Tandem Mass Spectrometry method was used to quantify several oxidized lipids. These methods were used to distinguish the difference among scalp tape strip extracts from non-dandruff, dandruff, and dandruff treatment subjects.

US Patent Application No.: 2010/267825 is directed to the treatment of skin damage and discloses a method of diagnosing skin damage based on the level of oxidized substances.

Jin-Ok Baek et al. (Arch Dermatol Res 2012, 304, 699-706) relates to the assessment of an imiquimod-induced psoriatic mouse model in relation to oxidative stress. The authors inter alia analyzed levels of Myeloperoxidase and superoxide dismutase-1.

Jang and Pezzuto (Cancer Letters, 1998, 134, 81-89) analyze the effects of resveratrol on 12-O-tetradecanoylphorbol-13-acetate-induced oxidative events and gene expression in mouse skin. Inter alia, evaluation of the potential of resveratrol to modulate TP-induced oxidative stress by assessing Myeloperoxidase activity was performed.
Kerr et al. (International Journal of Dermatology 2011, 50, 102-113) analyze epidermal changes associated with symptomatic resolution of dandruff, by evaluating biochemical markers of inflammation (IL-1α, IL-1RA, IL-8) and barrier integrity (keratin 1, 10, 11; involucrin; SC lipids; human serum albumin) in stratum corneum samples. Noninvasive sampling methods are important for biomarker applications in skin /scalp care. Tape strips have been successfully used for collection of small molecules and proteins associated with oxidative stress and oxidative damage from skin/scalp for subsequent analyses.

### SUMMARY OF THE INVENTION

The present invention is directed to a noninvasive method for diagnosing dandruff condition in a subject comprising: collecting a skin sample/epithelial cell sample from the subject; detecting a level of the biomarker Myeloperoxidase in the epithelial cell sample/skin cell sample; diagnosing the subject as having oxidative stress and/or oxidative damage based on the level of a detected biomarker, wherein there is a change in biomarker level when compared to a baseline level of biomarker; and thus diagnosing dandruff condition.
An aspect of the disclosure is directed to the noninvasive method, wherein diagnosing dandruff condition in a subject comprises: Applying an adhesive article to an epithelium of a mammal; Allowing for adherence of epithelial cells to the adhesive article; removing the adhesive article from the epithelium of the mammal; preparing the adhesive article using standard laboratory methods for extraction; extracting thebiomarker Myeloperoxidase from the epithelial cells adhered to said adhesive article; measuring the biomarkers from the epithelial cells adhered to said adhesive article; determining the amount of the biomarker in the epithelial cells as compared to a baseline sample following a treatment; and diagnosing the subject as having oxidative stress and/or oxidative damage based on the level of a detected biomarker, wherein there is a change in biomarker level when compared to a baseline level of biomarker; and thus diagnosing dandruff condition.

These and other features, aspects, and advantages of the present invention will become evident to those skilled in the art from a reading of the present disclosure.

### BRIEF DESCRIPTION OF THE DRAWINGS

Figure 1 is a graph showing comparison of myeloperoxidase levels in non-dandruff versus dandruff sufferers.
Figure 2 is a graph showing the change from baseline in myeloperoxidase levels after treatment with either an anti-dandruff shampoo or non-dandruff shampoo for dandruff sufferers.
Figure 3 is a graph showing normalization of myeloperoxidase levels in dandruff sufferers at baseline and after treatment with an anti-dandruff shampoo versus a non-dandruff population.
Figure 4 is a graph showing comparison of (±)-9-hydroxy-10E, 12Z-octadecadienoic acid and (±)-13-hydroxy-10E, 12Z-octadecadienoic acid (HODE) and Squalene Hydroperoxide levels in non-dandruff versus dandruff sufferers.
Figure 5 is a graph showing oxidized lipids change from baseline in dandruff sufferers after treatment with either an anti-dandruff shampoo or a non-dandruff shampoo.

### DETAILED DESCRIPTION OF THE INVENTION

While the specification concludes with claims which particularly point out and distinctly claim the disclosure, it is believed the present disclosurewill be better understood from the following description.

The present disclosure can comprise, consist of, or consist essentially of the essential elements and limitations of the disclosure described herein, as well any of the additional or optional ingredients, components, or limitations described herein.

All percentages, parts and ratios are based upon the total weight of the compositions of the present disclosure, unless otherwise specified. All such weights as they pertain to listed ingredients are based on the active level and, therefore; do not include carriers or by-products that may be included in commercially available materials.

The components and/or steps, including those, which may optionally be added, of the various aspects of the disclosure, are described in detail below.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more"

Herein, "comprising" means that other steps and other ingredients which do not affect the end result can be added. This term encompasses the terms "consisting of' and "consisting essentially of". The compositions and methods/processes of the present disclosure can comprise, consist of, and consist essentially of the essential elements and limitations of the disclosure described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.
As used herein, the term "differential level" of a biomolecule may include any increased or decreased level. In one aspect, differential level means a level that is increased by: at least 5%; by at least 10%; by at least 20%; by at least 30%; by at least 40%; by at least 50%; by at least 60%; by at least 70%; by at least 80%; by at least 90%; by at least 100%; by at least 110%; by at least 120%; by at least 130%; by at least 140%; by at least 150%; or more. In another
aspect, differential level means a level that is decreased by: at least 5%; by at least 10%; by at least 20%; by at least 30%; by at least 40%; by at least 50%; by at least 60%; by at least 70%; by at least 80%; by at least 90%; by at least 100%. A biomolecule is expressed at a differential level that is statistically significant (i.e., a p-value less than 0.2 (two-sided) as determined using, either Student T-test, Welch's T-test or Matched Pair T-test.

The term 'skin' means the outer covering of a vertebrate animal, consisting of two layers of cells, a thick inner layer (the dermis) and a thin outer layer (the epidermis). The epidermis is the external, nonvascular layer of the skin. It is made up, from within outward, of five layers of EPITHELIUM: (1) basal layer (stratum basale epidermidis); (2) spinous layer (stratum spinosum epidermidis); (3) granular layer (stratum granulosum epidermidis); (4) clear layer (stratum lucidum epidermidis); and (5) horny layer (stratum corneum epidermidis).

The term "sample" refers to any preparation from skin or epidermis of a subject.

The term "noninvasive" means a procedure that does not require insertion of an instrument or device through the skin or a body orifice for diagnosis or treatment.

The term "adhesive device" means a device used for the removal of the skin's epidermal layer by using an adhesive or an adhesive material on a substrate. For example, skin samples with adhesive tapes such as D-Squame® (polyacrylate ester adhesives; CuDerm; Dallas TX), Durapor, Sebutape™ (acrylic polymer films; CuDern; Dallas, TX), Tegaderm™, Duct tape (333 Duct Tape, Nashua tape products), Scotch® Tape (3M Scotch 810, St. Paul, Minn.), Diamond™ (The Sellotape Company; Eindhoven, the Netherlands), Sentega™ (polypropylene tape, Sentega Eiketten BV, Utrecht, The Netherlands) may be used. The adhesive may be any of the commonly used pressure-sensitive-type adhesives or those which solidify quickly upon skin content (such as cynaoacylates). The adhesives may be on flexible or solid backings to make sampling easier. A constant pressure device (e.g. Desquame Pressure Instrument, CuDerm; Dallas, TX) can be used to apply pressure to the adhesive device during sampling.

Samples from a tissue may be isolated by any number of means well known in the art. Invasive methods for isolating a sample include the use of needles, for example during blood sampling, as well as biopsies of various tissues, blistering techniques and laser poration. Due to the invasive nature of these techniques there is an increased risk of mortality and morbidity. Further, invasive techniques can inadvertently impact the state of the skin, which could lead to inaccurate or false results. Even further, invasive techniques are difficult to execute on a large population. The invasive technique may result in discomfort to the participant and may provide a greater potential for infection or other side effects. The present disclosure provides a noninvasive method for measuring biomarkers of oxidative stress and oxidative damage from the skin.

The term "objectively" means without bias or prejudice. Alternatively, any expert or self-assessments are inherently "subjective."

The term "normalization" and/or 'normalized" means the degree to which a population of dandruff sufferers approach a state of normal population.

The term "standardization" and/or "standardized" means biomarker values expressed relative to the amount of protein measured on the corresponding adhesive or adhesive article in the case of myeloperoxidase. In the case of oxidized lipids the standardization means the value of oxidized lipid is expressed relative to the corresponding non-oxidized parent lipid. A non-limiting example would be ng oxidized lipid/ ng parent lipid or pg myeloperoxidase/µg soluble protein.

The term "baseline" means information gathered at the beginning of a study from which variations found in the study are measured.

In a further aspect of the disclosure, there is a number of Alternative "Noninvasive" Sampling Methods that may be used.

Sebutape™: This is a noninvasive approach in that Sebutape™ (acrylic polymer film; CuDerm; Dallas, TX) is only very mildly adhesive and may be applied to and removed from even visibly inflamed skin without causing discomfort. Biomarkers recovered/assayed by this technique have included proteins (e.g., cytokines), peptides (e.g., neuropeptides), and small molecules (lipids) Historically, this tape is manufactured and sold for sebum collection and can, therefore, be useful for lipid analysis.

D-Squame®: D-Squame® tape is a polyacrylate ester adhesive also manufactured by CuDerm. It may be used to recover the same biomarkers as Sebutape™ but also removes certain epidermal structural proteins (e.g., keratins, involucrin). It has also been used to recover cortisol and serum albumin as systemic inflammatory markers, and small molecules (histamine) and stratum corneum lipids.

Cup Scrubs: Cup scrubs extract proteins directly from the surface of the skin, usually in the presence of buffer, a nonionic surfactant or an organic solvent (ex. ethanol). Cup scrubs are primarily used for recovery of soluble biomarkers such as cytokines, but can also be used to recover small organic molecules. Many more cytokines can be recovered and quantified from cup scrubs than from tape strips. This could be due to several reasons. (a) Due to the presence of detergents and their liquid nature, cup scrubs most likely sample a different protein population than do tape strips. (b) With cup scrubs, cytokines do not have to be further extracted after sample collection since they already are in solution.

Hair plucks: Plucking hairs is the process of removing human or animal hair by mechanically pulling the item from the owner's body usually with tweezers. The follicular region of the hair pluck is extracted usually in the presence of buffer and a nonionic surfactant for recovery of soluble protein biomarkers such as cytokines, and can also be extracted with an organic solvent to recover small organic molecules like lipids.

Animal (i.e. Dog) Collection Method: D-Squame®: D- Squame™ tape samples are collected on dogs' skin via parting their fur (without shaving). A variety of biomarkers related to skin inflammation, differentiation and barrier integrity can be analyzed from the tapes including total protein, soluble protein, skin multiple analyte profile (skin MAP), skin cytokines and stratum corneum lipids (ceramides, cholesterol, fatty acids).

In an aspect of the disclosure, the present invention provides a method and analysis for noninvasively obtaining a sample for use in isolating Myeloperoxidase. Also disclosed is a respective method for use in isolating oxidized lipids.

In an aspect, the use of an adhesive device can be used to achieve such sampling.
In preparation for such a sampling study for a dandruff sampling, at a baseline visit, a qualified screening grader will complete adherent scalp flaking score (ASFS) grading for each subject and the highest flaking octant will be identified for tape strip sampling. The highest flaking octant will be sampled at baseline and various time points. Tape strips samples will be collected from each subject at each time point.

The tape strip sampling is repeated additional times, as needed, at the same site placing each D-Squame® tape disc on top of the prior sampled area. The D-Squame® tapes after sample collection are placed into the appropriately labeled wells in a labeled plate.

Following the sampling, an extraction and quantitation procedure is conducted. Quantitation of myeloperoxidase and oxidized lipids from extracts of D-Squame® Tape Samples can be conducted via analysis by either antibody-based immunoassay or by LC/MS/MS. In this instance, the sample extraction in preparation for antibody based analysis or LC/MS/MS analysis was performed.

For the Myeloperoxidase method, appropriate standard extraction buffers are added to each collection tube and then extracted on ice using sonication for 30 min. Each extract solution is isolated from the tape strip and an aliquot of each sample is placed into a specified position of a 96-well polypropylene plate. Aliquots of the extracts of D-Squame® Tape samples are then supplemented with conventional reagents, such as albumin, to help prevent loss of analytes to the walls of labware, transferred into 96-well polypropylene deep well plates and frozen at -80°C for myeloperoxidase analysis. A separate aliquot is not supplemented with reagents and is analyzed for soluble protein using a BCA™ Protein Assay Kit, Pierce catalog #23227.

Following the extraction process, Myeloperoxidase standards and controls can be prepared by conventional methods. Myeloperoxidase will be quantitated with a myeloperoxidase immunoassay kit from Mesoscale Discovery. The result can be reported as the amount of Myeloperoxidase/tape strip or the result can be standardized by dividing by the amount of myeloperoxidase by the amount of the protein that was also found in the tape strip extract. The protein method has been described separately. Data analysis is conducted by standard statistical methods and calculations.

Quantitation of oxidized lipids from extracts of the adhesive article, tape strips, can be conducted using gradient reversed-phase high performance liquid chromatography with tandem mass spectrometry (HPLC/MS/MS).

Tape strips (single or multiple tape strips) obtained from the scalp of human subjects are placed into individual polypropylene amber vials or glass amber vials, and then extracted with extraction solvent (methanol with 0.1% butylated hydroxytoluene, w/v) using vortexing for 10 min. The standards and the extracts of the scalp tape strips are analyzed using gradient reversed-phase high performance liquid chromatography with tandem mass spectrometry (HPLC/MS/MS). Analytes (oxidized or non-oxidized lipids) listed in Table 1 and the ISTDs are monitored by positive ion electrospray (ESI). A standard curve is constructed by plotting the signal, defined here as the peak area ratio (peak area analyte /peak area ISTD) or peak area analyte only, for each standard versus the mass of each analyte for the corresponding standard. The mass of each analyte in the calibration standards and human scalp extract samples are then back-calculated using the generated regression equation. The result can be reported as the mass of oxidized lipid/tape strip or the result can be standardized by dividing by the amount of oxidized lipid by the amount of the corresponding parent non-oxidized lipid that was also found in the tape strip extract. Additionally results could be reported by standardizing the amount of oxidized lipid by the amount of corresponding protein found in the tape strip extract. Standardization could also be done by collecting the cells removed, drying them and weighing them.

**Table 1 - Analytes**

| |
|---|
| 9/13-HODE |
| (±)-9-hydroxy-10E, 12Z-octadecadienoic acid and (±)-13-hydroxy-10E, 12Z-octadecadienoic acid (HODE) |
| 9/13-HpODE |
| (±)-9-hydroperoxy-10E, 12Z-octadecadienoic acid and (±)-13-hydroperoxy-10E, 12Z-octadecadienoic acid (HODE) |
| CH-OOH |
| Cholesterol Hydroperoxide |
| SQ-OOH |
| Squalene Hydroperoxide |
| Oxidosqualene |
| 5α, 6α-epoxy-Chol |
| 5α, 6α-epoxy-cholesterol |
| 4β-OH-Chol |
| 4β-hydroxycholesterol |
| 7β-OH-Chol |
| 7β-hydroxycholesterol |
| Linoleic acid |
| Cholesterol |
| Squalene |

### Methodology Extension

Although the exact procedure used is described above, there are a number of alternate approaches that could be taken for a number of the steps outlined above that are logical extensions. The extraction solvents employed for isolating Myeloperoxidase and oxidized lipids from the tape strip can be any appropriate aqueous, organic or organic/aqueous mixture that provides a suitable recovery. LC/MS/MS and antibody-based immunoassays are generally recognized as the state-of-the-art approaches for the quantitative analysis of organic molecules in biological matrices due to their high selectivity and sensitivity. However, any analytical technique and or other approach providing the required sensitivity and selectivity could be employed. For example, other methods for assessing biomolecules have been employed including: capillary electrophoresis, supercritical fluid and other chromatographic techniques and/or combinations thereof. Similarly, instrumental approaches without separation techniques have also been employed including nuclear magnetic resonance spectroscopy, mass spectrometry, electrochemical and fluorometric assays. Additionally, ligand binding approaches such competitive and non-competitive enzyme linked immunosorbent assays (ELISAs) and radioimmunoassay (RIA) or other labeling schemes have also been employed. Enzyme-based assays have a long history of use in the analysis of proteins. Bioassay using either cell-based or tissue-based approaches could have also been used as the means of detection. In an aspect of the disclosure, quantitation of biomarkers of oxidative stress and oxidative damage from hair plucks can be carried out with the same basic extraction and analysis methods as used for tape strip samples.

### Protein Determination of Tape Strip Extracts:

The level of myeloperoxidase on tape strip samples of skin measured using a suitable methodology described above can be standardized using amount of protein found in the tape strip extract. Standardization is done by dividing the amount of myeloperoxidase by the amount of protein in the tape strip extract.

The amount of protein in the tape strip extract or an equivalent matrix that was used to determine the Myeloperoxidase level on skin can be determined using variety of protein determination methods described in the literature. Examples of such methods include total nitrogen determination, total amino acid determination and protein determination based on any colorimetric, flurometric, luminometric methods. These methods may or may not involve further sample preparation of the tape strip extract prior to protein determination. A non-limiting example of a specific method for protein determination in the tape strip extract is given below. A comprehensive review of protein determination methods, their applicability and limitations are described in the Thermo Scientific Pierce Protein Assay Technical Handbook that can be downloaded from the following link. www.piercenet.com/Files/1601669_PAssayFINAL_Intl.pdf. Further information related to protein determination can be found at Redinbaugh, M.G. and Turley, R.B. (1986). Adaptation of the bicinchoninic acid protein assay for use with microtiter plates and sucrose gradient fractions. Anal.Biochem. 153, 267-271.

Adhesive tapes sampled from human skin will be extracted and analyzed for protein content using the BCA™ Protein Assay Kit (Pierce). The tape strips sampled from human skin will be extracted with a conventional extraction buffer. Following extraction, aliquots of the tape extracts will be transferred into 96-well polypropylene deep well plates and stored at 2 - 8°C for protein determination.

The BCA™ Protein Assay Kit is based on the reduction of Cu²⁺ to Cu¹⁺ by proteins in an alkaline medium coupled with the sensitive and selective colorimetric detection of Cu⁺¹ by bicinchoninic acid (BCA). The purple-colored reaction product, formed by chelation of 2 molecules of BCA with one Cu¹⁺ ion, exhibits strong absorbance at a wavelength of 562 nm. The optical density (OD) is measured using a microplate reader. Increasing concentrations of Bovine Serum Albumin (BSA), expressed in micrograms per milliliter (µg/mL), are used to generate a calibration curve in the assay. Appropriate assay QC's prepared from the BSA stock solution will be used to monitor assay performance during sample analysis.

In an alternative aspect of the disclosure, protein determination can be done direct measurement of protein on an adhesive or an adhesive article such as protein measurement with a SquameScan® 850A (CuDerm Corporation, Dallas, Texas).

In a further aspect of the disclosure, additional oxidative stress markers (in addition to unsaturated fatty acid hydroperoxides/hydroxides, cholesterol hydroperoxides/hydroxides and squalene hydroperoxide/oxide/hydroxides) may include the following:

### Heat shock protein (Hsp) 27

The cytoprotective properties of Hsp27 result from its ability to modulate reactive oxygen species and to raise glutathione levels.

Heat shock protein 27 (HSP27) belongs to the small molecular weight heat shock protein (HSP) family (12-43 kDa). HSP27 and other members of the small HSP family share a conserved c-terminal domain, the α-crystallin domain, which is identical to the vertebrate eye lens α-crystallin [1]. HSP27 was initially characterized in response to heat shock as a protein chaperone that facilitates the proper refolding of damaged proteins. Continued investigation of HSP27 revealed that the protein responds to cellular stress conditions other than heat shock; for example oxidative stress and chemical stress. During oxidative stress, HSP27 functions as an antioxidant, lowering the levels of reactive oxygen species (ROS) by raising levels of intracellular glutathione and lowering the levels of intracellular iron.

### Oxidative modification of Proteins

The oxidation of proteins in biological systems occurs by spontaneous autoxidation of cysteinyl thiols, interactions of proteins with reactive oxygen species (ROS) and by deliberate and controlled reactions catalyzed by oxidases. Reaction of proteins with ROS can result in oxidation of cysteine, methionine, tyrosine, phenylalanine and tryptophan residues. Methione oxidation is monitored by determining methionine sulfoxide, oxidation of tyrosine can by the amount of dityrosine formed, oxidation of phenylalaninine by the formation of o-tyrosine and m-tyrosine, oxidation of tryptophan residues is followed by monitoring N-formylknurenine, kynurenine and /or quinolinic acid. Also, the covalent and oxidative modification of albumin cys34 residue has been suggested as a specific biomarker of mild oxidative stress. Usually, these protein modification adduct residues are determined after exhaustive enzymatic hydrolysis or chemical digestion.

Proteins can be modified via oxidative pathways involving the formation of protein carbonyl groups mainly formed from lysine, proline and arginine residues. Lysine forms 2-aminoadipic semialdehyde (AASA) via oxidative deamination and glutamic semialdehyde (GSA) is formed by oxidation of proline and arginine residues. The AASA and GSA can be detected after reduction to give 6-hydroxy-2-aminocaproic acid and 5-hydroxy-2-aminovaleric acid, respectively. Protein carbonylation can also be determined by ELISA based approaches following derivitization with 2,4-dintirophenylhydrazine.

Additionally, proteins can be modified due to oxidative pathways via reaction with α,β-unsaturated alkenals formed from the oxidation of polyunsaturated fatty acids (see Reaction Products of - unsaturated alkenals with Protein and Mercapturic Acid Pathway) and by the formation of early glycation adducts (EGA) and advanced glycation products (AGEs) with sugars (see EGA and AGEs).

### DNA Oxidation and Hydroxylated nucleotides¹

DNA damage is generally one measure of oxidative stress with the main cause due to free radical damage caused by endogenous reactive oxygen species (ROS) Oxidative damage to intact DNA can be measured using the COMET assay. Oxidative damage can be measured by monitoring a variety of hydroxylated nucleotides including 8-hydroxydexoygaunosine (8OHdG) which is also referred to as 8-oxodeoxyguanosine (8-oxodG), 4,6-diamino-5-form-amidopyrimidine (FapyAde) and 2,6-diamino-4-hydroxy-5-formamidopyrimidine (FapyGua).

### Isoprostanes¹

Isoprostanes are a series of prostaglandin-like isomers formed from the free-radical catalyzed oxidation of the polyunsaturated fatty acids ((PUFAs) such as arachidonic acid (AA) and the omega-3 eicosapentaenoic acid (EPA), typically the oxidation of PUFAs occurs to the phospholipid form. Isoprostanes derived from AA oxidation result in an F-type prostane rings (referred to as F₂-Isoprostanes) and give rise to the 5-F₂-series IsoP, 8-F₂-series IsoP, 12-F₂-series IsoP and 15-F₂-series IsoPs8-series. Similarly, D/E-ring and A/J-ring isoprostanes are also formed are formed from AA also. Isoprosanes of the F₃-family are similarly formed from EPA and give rise to 5-F₃-series, 8-F₃-series, 11-F₃-series, 12-F₃-series, 15-F₃-series and 18-F₃-series Isoprostanes.

### α,β-unsaturated aldehydes¹

Oxidation of polyunsaturated fatty acids often occurs in response to oxidative stress resulting in a radical driven formation of fatty acid hydroperoxides which can undergo further reaction to give a wide diversity of α,β-unsaturated alkenals in biological systems, such as malondialdehyde, acrolein, crotonaldehyde, 4-hydroxynonenal, 4-hydroxyhexenal and 4-oxo-nonenal. These α,β-unsaturated alkenals have been followed as biomarkers of oxidative damage.

### Reaction Products of α,β-unsaturated alkenals with Protein and Mercapturic Acid Pathway

The α,β-unsaturated alkenals are reactive electrophiles and form products with a number of nucleophilic compounds including covalent protein adducts referred to as advanced lipoxidation end-products (ALE) such as hexanoyl-lysine, hexanoyl-histidine, Ne-(3-methylpyridinium)lysine and with mercapturic acid compounds (glutathione, cysteine and mercapturic acid) to form thioethers (ex. 1,4-dihydronone mercapturic acid, 3-hydroxypropylmercapturic acid and carboxyethylmercapturic acid).

### Early Glycation Adducts (EGA) and Advanced Glycation End Products (AGE)

Glycation of proteins is a non-enzymatic complex series of parallel and sequential reactions collectively called the Maillard reaction and occurs in all tissues and body fluids. Glycation adducts can be formed by the reaction of proteins with glucose and reactive α-oxoaldehydes such as glyoxal, methylglyoxal and 3-deoxyglucosone and other saccharide derivatives. Early stage reactions in glycation lead to the formation of fructosyl-lysine and N-terminal amino acid residue-derived fructosamines and are referred to as Early Glycation Adducts (EGA). Later stage reactions form stable end stage adducts called advanced glycation end products such as monolysyl adducts (carboxymethyl lysine (CML), carboxyethyl lysine (CEL) and pyrraline), monovalyl adducts (carboxymethyl valine (CMV) and carboxyethylvaline (CEV)), hydroimidazolones, bis(lysyl) imidazolium crosslinks (GOLD, MOLD, DOLD) and pentosidine derived from a cross link of lysine and arginine. The EGAs and AGEs are released when proteins are degraded by proteolysis or when proteins are degraded by chemical lysis. The formation and accumulation of AGEs have been implicated in the progression of age-related diseases. Research over the last 20 years has implicated AGEs in most of the diseases associated with aging. CML may be a general marker of oxidative stress and long term damage to protein in aging, atherosclerosis, and diabetes.

### Antioxidants as Biomarkers of Oxidative Stress¹

Endogenous antioxidants play a key defense role in controlling oxidative damage caused by radical's mechanisms. Key endogenous antioxidants include ascorbic acid (AsA), glutathione (GSH), α-tocopherol and Coenzyme Q 10 (CoQ). Changes in the levels of these endogenous redox (oxidized and reduced forms) antioxidants can be used as a measure of oxidative stress. (**¹**) Biomarkers for Antioxidant Defense and Oxidative Damage: Principles and Practical Applications; G. Aldini, K-J Yeum, E. Niki and R.M. Russell, eds, Wiley-Blackwell, Iowa, 2010.

### EXAMPLES

### Basic Procedure for Assessment of Oxidative Stress and Oxidative Damage

Subjects are evaluated by a qualified grader to establish their scalp status. Dandruff subjects are identified by their level of visible flaking as assessed by a qualified grader. Non-dandruff healthy scalp subjects are evaluated at baseline to establish normalized levels of each biomarker. Subjects were identified by a qualified grader in two separate clinical studies; study #1 and study #2. In Study #1, there are 60 non-dandruff subjects that are evaluated at baseline for MPO levels. There are 119 dandruff sufferers placed on a 1% ZPT containing anti-dandruff shampoo, 115 dandruff sufferers placed on a 1% selenium sulfide containing anti-dandruff shampoo and 60 dandruff subjects placed on a non-dandruff shampoo that did not contain an anti-dandruff active. They are evaluated for MPO levels at baseline, and after 1 week, 2 weeks and 3 weeks of product usage. In Study #2 there are 30 non-dandruff subjects evaluated at baseline for oxidized lipids. There were 60 dandruff subjects placed on an anti-dandruff shampoo and 60 dandruff subjects placed on a non-dandruff shampoo which contained no anti-dandruff active. They were evaluated for oxidized lipids at baseline and again after 3 weeks of product usage.

Subjects undergo a two week washout period with a conventional non-dandruff shampoo without conditioning agents prior to a treatment period with an anti-dandruff shampoo. Dandruff subjects undergo a three week treatment period with an anti-dandruff shampoo (a shampoo composition containing zinc pyrithione, or selenium sulfide) or a non-dandruff shampoo with no anti-dandruff active, tape strip samples are collected from the highest flaking octant as determined at the baseline visit by qualified grader. For dandruff subjects, scalp tape strips are taken at baseline and after product treatment. For non-dandruff subjects, scalp tape strips are taken at baseline only. Tapes are kept at -80°C until extracted. A dandruff-involved site is sampled by parting the hair, applying a D-Squame® tape (CuDerm Corporation), and rubbing the tape, as needed. For the non-dandruff subjects, a flake free site is sampled. The tapes are placed into a pre-labeled 12 well culture dish for storage.

Samples are extracted with a conventional extraction buffer and sonicated on ice. Aliquots of the extracts of D-Squame® Tape samples are then transferred into pre-labeled polypropylene collection tubes and frozen at -80°C for analysis for MPO and for the oxidized lipids. A separate aliquot is specified for soluble protein analysis using a BCA™ Protein Assay Kit, Pierce catalog #23227 for the MPO extracts.

Following extraction, the samples are analyzed for Myeloperoxidase and oxidized lipids by antibody-based immunoassay or by LC/MS/MS. The relative quantitated values for the compounds were then standardized to the amount of soluble protein in the extract as determined by the BCA protein assay as outlined above for myeloperoxidase or by amount of the corresponding parent non-oxidized lipid for the oxidized lipids. Analysis is conducted at baseline and after treatment for samples from the anti-dandruff shampoo treatment group and for baseline only for the non-dandruff group. Matched pair T-test was used to analyze the differences among the non-dandruff (healthy), dandruff baseline and dandruff treated subjects. Dandruff treated subjects were compared to non-dandruff to determine degree of normalization upon treatment with an anti-dandruff product.

### RESULTS SUMMARY

Statistical Analysis of Data: data was collected on dandruff subjects at both baseline and after treatment) and non-dandruff subjects at baseline. Given the wide variability of analyte levels between subjects as well as within subject (baseline -> treatment), it is more efficient to transform the data to the log10 scale before analysis. Statistical analysis was carried out on the equivalent log10 Ratio values (e.g., log10(Week 3/Baseline)). Final reported results are then converted back to their original scale (or % change from baseline values), for ease of interpretation. All statistical analyses were carried out using the SAS JMP (Version 7.0.2) software. A p-value of ≤ 0.20 (two-sided) was used to determine statistical significance.

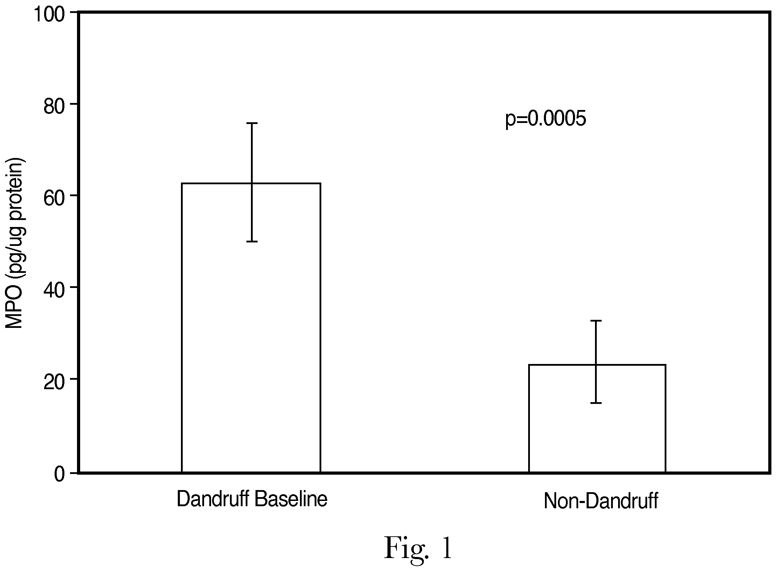

The data in Figure 1 demonstrates that Dandruff MPO levels were significantly higher than non-dandruff levels (p-value = 0.0005). MPO levels on tape strip samples of human scalp measured using a suitable methodology described above can be standardized using amount of protein found in the tape strip extract. MPO levels were determined from an independent non-dandruff group to establish normal skin MPO levels. Standardization of MPO levels is done by dividing the MPO level by the amount of protein in the tape strip extract.

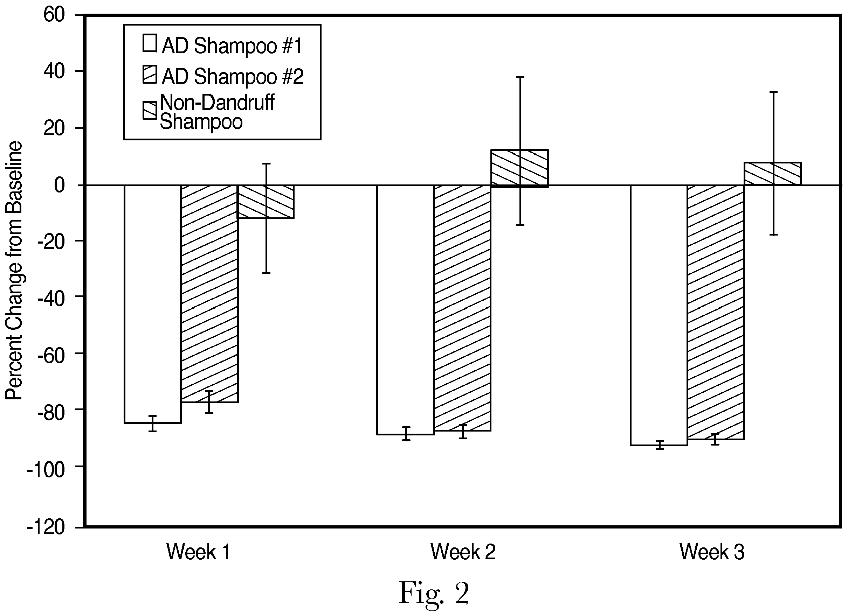

The data in Figure 2 demonstrates that there was a 77% reduction in MPO level following treatment with an anti-dandruff shampoo for one week, an 87% reduction after two weeks of treatment and an 89% reduction over a 3-week period of time versus baseline levels (p-values <0.0001). A reduction in MPO is consistent with an improvement as demonstrated by comparing levels to the non-dandruff population. The anti-dandruff shampoo demonstrated a significant improvement versus a non-dandruff shampoo at all treatment time points (p-values <0.0001). These MPO levels have been standardized by the amount of protein in the tape strip extract.

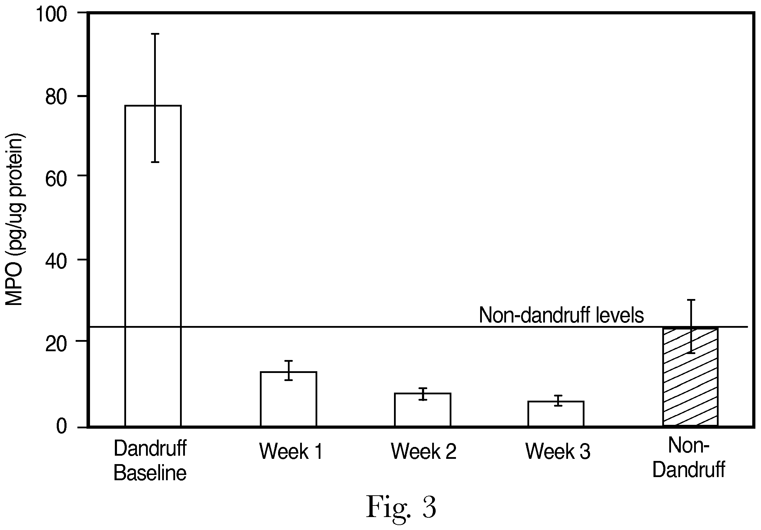

The data in Figure 3 demonstrates that the anti-dandruff treatment restored MPO levels to that of the non dandruff group as discussed in Figure 1 above. This is a useful means of communicating the benefit as the dandruff population desires a return to a normalized and/or healthy state.

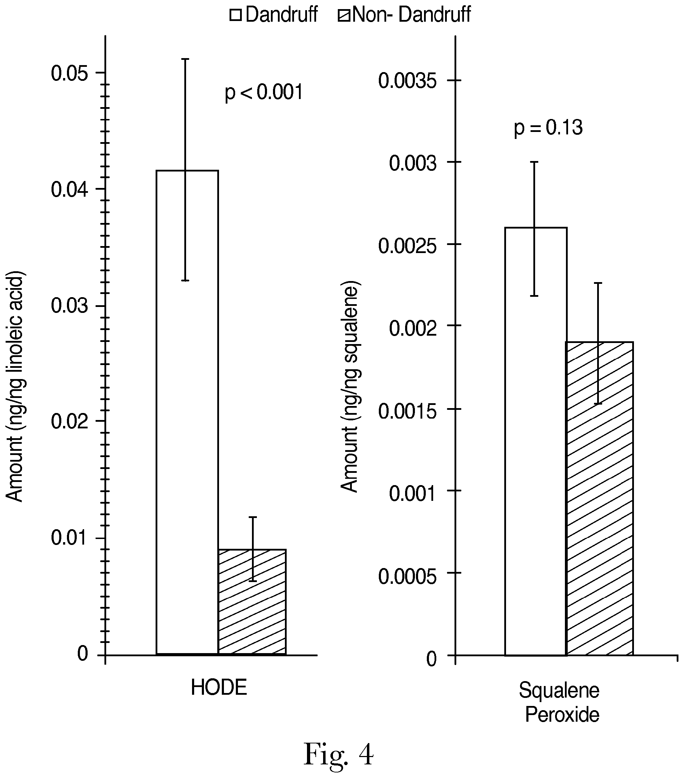

The data in Figure 4 demonstrates that Dandruff HODE levels were significantly higher than nondandruff levels (p-value < 0.001). The data in Figure 4 demonstrates that Dandruff SQOOH levels were significantly higher than non-dandruff levels (p-value = 0.13). Oxidized lipids on tape strip samples of human scalp measured using a suitable methodology described above can be standardized by dividing the oxidized lipid by its corresponding parent non-oxidized lipid in the tape strip extract. The level of HODE has been standardized by dividing the HODE level by the amount of linoleic acid in the tape extract and the SQOOH was standardized by dividing by the amount of squalene in the tape strip extract.

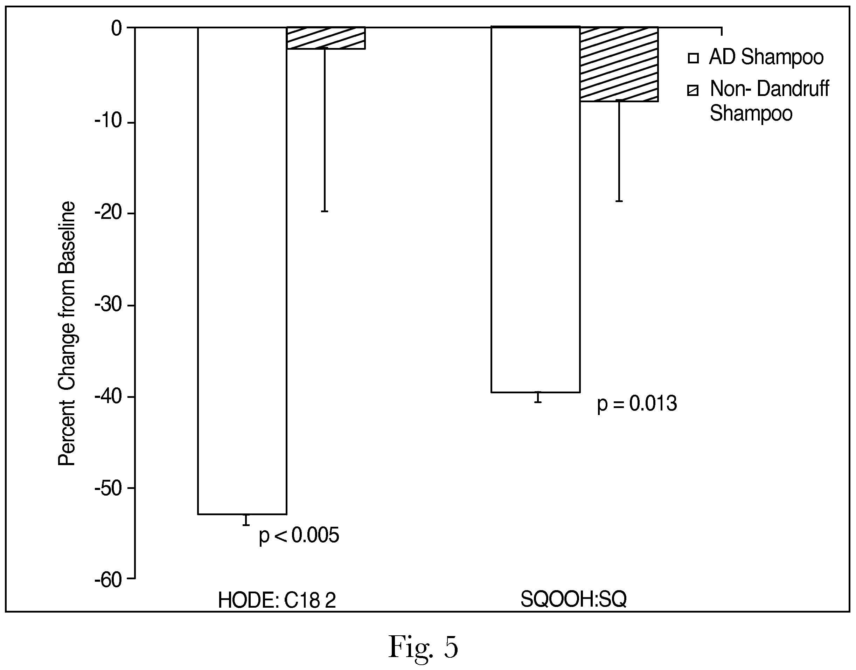

The data in Figure 5 demonstrates a significant reduction in standardized oxidized linoleic acid (HODE) as measured after 3 weeks of treatment with an anti-dandruff shampoo versus baseline (p-value <0.01). A reduction is consistent with an improvement as demonstrated by comparing levels to the non-dandruff population. The anti-dandruff shampoo demonstrated a significantly improvement versus a non-dandruff shampoo (p-value =0.005). The data in Figure 5 demonstrates a significant reduction in standardized squalene hydroperoxide as measured after 3 weeks of treatment with an anti-dandruff shampoo versus baseline (p-value <0.01). A reduction is consistent with an improvement as demonstrated by comparing levels to the non-dandruff population. The anti-dandruff shampoo demonstrated a significantly improvement versus a non-dandruff shampoo (p-value =0.013).

In particular aspects, such performance assessments can be advantageous for comparing a subject's skin health status before and after treatment with an anti-dandruff composition. For example, some anti-dandruff compositions may promote a faster speed of relief that will be experienced by the subject if the subject switches to and maintains a certain anti-dandruff composition or regimen. An assessment that allows objective measurement of particular skin health benefits of the subject could allow for the comparing of a subject's status before the treatment with a particular anti-dandruff composition or regimen and after the subject switches to the particular anti-dandruff composition or regimen. Additionally, such an assessment could be used as a supporting credentialing tool that supports particular skin health benefit claims that the particular treatment or regimen is promoting. For example, if a particular composition or regimen is promoting that it will decrease the symptoms of itch, an assessment as disclosed herein can be used to support the specific health benefit claim to show that the subject's discomfort from itch has decreased. Non-limiting elements of skin health that could be benefited by anti-dandruff compositions and regimens include itch, flaking, irritation, skin barrier integrity, dryness, oxidative stress and oxidative damage, self-defense, natural protection.

The results of many analyses may also be used as marketing or advertising information to promote the effectiveness of particular products, combinations of products, and techniques. Examples of advertising claims that could be placed on product packaging that might be substantiated by the present disclosure include, but are not limited to, establishment claims (e.g., "clinically proven" or "tests show"), before and after claims (e.g., "50% less dandruff after use"), monadic claims, comparative claims, factor-claims (e.g., "3X reduction in dandruff"), and prevention and treatment claims. For example, product packages may refer to an analysis and demonstrate objectively-proven effectiveness or comparisons of the product. Also, analysis data may be used in clinical information related to different regimen that may or may not by used in combination with different products or groups of products.

A further aspect includes wherein there is a change in standardized biomarker following application with a zinc pyrithione shampoo when compared to a baseline level of biomarker prior to the application. In another aspect, there is a change in standardized molecule biomarker following application with a selenium sulfide shampoo when compared to a baseline level of biomarker prior to the application. In a further aspect, there may be a measure of biomarker that establishes an improvement of at least 5% in dandruff condition compared to a normal population following treatment. In a further aspect, there may be an improvement of at least 10%, at least 20%, at least 30%, at least 40%, at least 50%, at least 60%, at least 70%, at least 80%, at least 90% and further, 100% improvement in dandruff condition compared to a normal population following treatment. In another aspect, there may be at least a 5% difference between dandruff and non-dandruff (no treatment). Further, there may be at least 10% difference between dandruff and non-dandruff, at least 20%, at least 30%, at least 40%, at least 50%, at least 60%, at least 70%, at least 80%, at least 90% between dandruff and non-dandruff and further, 100% difference or greater between dandruff and non-dandruff.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A noninvasive method for diagnosing dandruff condition in a subject comprising:
detecting a level of the biomarker Myeloperoxidase in an epithelial cell sample/skin cell sample obtained from the subject; and
diagnosing the subject as having oxidative stress and/or oxidative damage based on the level of a detected biomarker, wherein there is a change in biomarker level when compared to a baseline level of biomarker; and thus diagnosing dandruff condition.

2. The method according to any preceding claims wherein collection of skin sample is selected from the group consisting of adhesive articles, hair plucks, skin wash and mixtures thereof.

3. The method according to any preceding claims wherein the level of one or more further biomarkers is detected, wherein the one or more further biomarkers is/are selected from the group consisting of (±)-9-hydroxy-10E, 12Z-octadecadienoic acid and (±)-13-hydroxy-10E, 12Z-octadecadienoic acid (HODE), squalene hydroperoxide, heat shock protein 27 (HSP27), oxidative modification of proteins, DNA oxidation and hydroxylated nucleotides, isoprostanes, α,β-unsaturated aldehydes, reaction products of α,β-unsaturated alkenals with protein and mercapturic acid pathway, early glycation adducts (EGA) and advanced glycation end products (AGE), antioxidants as biomarkers of oxidative stress and mixtures thereof.

4. The noninvasive method according to any preceding claims, wherein the method comprises:
a) applying an adhesive article to an epithelium of a mammal;
b) allowing for adherence of epithelial cells to the adhesive article;
c) removing the adhesive article from the epithelium of the mammal;
d) preparing the adhesive article using standard laboratory methods for extraction;
e) extracting the biomarker Myeloperoxidase from the epithelial cells adhered to said adhesive article;
f) measuring the biomarkers from the epithelial cells adhered to said adhesive article;
g) determining the amount of the biomarker in the epithelial cells as compared to a baseline sample following a treatment; and
h) diagnosing the subject as having oxidative stress and/or oxidative damage based on the level of a detected biomarker, wherein there is a change in biomarker level when compared to a baseline level of biomarker; and thus diagnosing dandruff condition.

5. The method according to any preceding claims, wherein the level of the detected biomarker is standardized by dividing the biomarker by an amount of protein on the adhesive article.

6. The method according to any preceding claims wherein the epithelium comprises stratum corneum.

7. The method according to any preceding claims wherein there is a change from baseline in standardized biomarker following application with an antifungal hair treatment, when compared to a baseline level of biomarker prior to the application.

8. The method according to any preceding claims wherein there is at least a 40% reduction in standardized biomarker over a 3-week period of time following application with an antidandruff shampoo when compared to a baseline level of biomarker prior to the application.

9. The method according to any preceding claims wherein there is an 87% reduction in Myeloperoxidase standardized biomarker over a 3-week period of time following application with an anti-dandruff shampoo when compared to a baseline level of Myeloperoxidase biomarker prior to the application.

10. The method according to any preceding claims wherein there is a change in standardized biomarker following application with a zinc pyrithione shampoo when compared to a baseline level of biomarker prior to the application.

11. The method according to any preceding claims wherein there is a change in standardized biomarker following application with a selenium sulfide shampoo when compared to a baseline level of biomarker prior to the application.

12. The method according to any one of claims 7-11 wherein there is improvement in dandruff condition compared to a normal population.

13. The method according to one of claims 7-12 wherein there is a 100% return in dandruff condition for Myeloperoxidase after treatment.

14. The method according to any preceding claims wherein there is at least a 5% difference between dandruff and non-dandruff.

## Patentansprüche

1. Nicht-invasives Verfahren zur Diagnose einer Schuppenerkrankung bei einem Probanden, umfassend:
Erfassen eines Niveaus des Biomarkers Myeloperoxidase in einer Epithelzellprobe/Hautzellprobe, die von dem Probanden erhalten wurde; und
Diagnostizieren von oxidativem Stress und/oder oxidativem Schaden bei dem Probanden auf Grundlage des Niveaus eines erfassten Biomarkers, wobei eine Änderung des Biomarkerniveaus im Vergleich zu einem Ausgangsniveau des Biomarkers vorliegt; und damit Diagnostizieren der Schuppenerkrankung.

2. Verfahren nach einem der vorstehenden Ansprüche, wobei das Nehmen der Hautprobe ausgewählt ist aus der Gruppe, bestehend aus Klebstoffartikeln, Haarausrissen, Hautwäsche und Mischungen davon.

3. Verfahren nach einem der vorstehenden Ansprüche, wobei das Niveau eines oder mehrerer weiterer Biomarker erkannt wird, wobei der eine oder die mehreren weiteren Biomarker ausgewählt ist/sind aus der Gruppe bestehend aus (±)-9-Hydroxy-10E, 12Z-Octadecadiensäure und (±)-13-Hydroxy-10E, 12Z-Octadecadiensäure (HODE), Squalenhydroperoxid, Hitzeschockprotein 27 (HSP27), oxidativer Modifikation von Proteinen, DNA-Oxidation und hydroxylierten Nukleotiden, Isoprostanen, α,β-ungesättigten Aldehyden, Reaktionsprodukten von α,β-ungesättigten Alkenalen mit Protein- und Mercaptursäureweg, frühen Glykierungsaddukten (EGA) und fortgeschrittenen Glykierungsendprodukten (AGE), Antioxidantien als Biomarker für oxidativen Stress und Mischungen davon.

4. Nicht-invasives Verfahren nach einem der vorstehenden Ansprüche, wobei das Verfahren Folgendes umfasst:
a) Auftragen eines Klebstoffartikels auf ein Epithel eines Säugetiers;
b) Ermöglichen des Anhaftens von Epithelzellen an dem Klebstoffartikel;
c) Entfernen des Klebstoffartikels von dem Epithel des Säugetiers;
d) Vorbereiten des Klebstoffartikels mittels Standard-Laborverfahren zur Extraktion;
e) Extrahieren des Biomarkers Myeloperoxidase aus den Epithelzellen, die an dem Klebstoffartikel haften;
f) Messen der Biomarker aus den Epithelzellen, die an dem Klebstoffartikel haften;
g) Bestimmen der Menge des Biomarkers in den Epithelzellen im Vergleich zu einer Ausgangsprobe nach einer Behandlung; und
h) Diagnostizieren von oxidativem Stress und/oder oxidativem Schaden bei dem Probanden auf Grundlage des Niveaus eines erfassten Biomarkers, wobei eine Änderung des Biomarkerniveaus im Vergleich zu einem Ausgangsniveau des Biomarkers vorliegt; und damit Diagnostizieren der Schuppenerkrankung.

5. Verfahren nach einem der vorstehenden Ansprüche, wobei das Niveau des erkannten Biomarkers durch Dividieren des Biomarkers durch eine Proteinmenge an dem Klebstoffartikel standardisiert wird.

6. Verfahren nach einem der vorstehenden Ansprüche, wobei das Epithel Stratum corneum umfasst.

7. Verfahren nach einem der vorstehenden Ansprüche, wobei eine Änderung vom Ausgangswert des standardisierten Biomarkers nach Anwendung einer antimykotischen Haarbehandlung im Vergleich zu einem Ausgangsniveau des Biomarkers vor der Anwendung vorliegt.

8. Verfahren nach einem der vorstehenden Ansprüche, wobei eine Verringerung von mindestens 40 % des standardisierten Biomarkers über einen Zeitraum von 3 Wochen nach Anwendung eines Antischuppen-Shampoos im Vergleich zu einem Ausgangsniveau des Biomarkers vor der Anwendung vorliegt.

9. Verfahren nach einem der vorstehenden Ansprüche, wobei eine Verringerung von 87 % des standardisierten Biomarkers Myeloperoxidase über einen Zeitraum von 3 Wochen nach der Anwendung eines Antischuppen-Shampoos im Vergleich zu einem Ausgangsniveau des Biomarkers Myeloperoxidase vor der Anwendung vorliegt.

10. Verfahren nach einem der vorstehenden Ansprüche, wobei eine Änderung des standardisierten Biomarkers nach Anwendung eines Zink-Pyrithion-Shampoos im Vergleich zu einem Ausgangsniveau des Biomarkers vor der Anwendung vorliegt.

11. Verfahren nach einem der vorstehenden Ansprüche, wobei eine Änderung des standardisierten Biomarkers nach Anwendung eines Selensulfid-Shampoos im Vergleich zu einem Ausgangsniveau des Biomarkers vor der Anwendung vorliegt.

12. Verfahren nach einem der Ansprüche 7-11, wobei eine Verbesserung der Schuppenerkrankung im Vergleich zu einem Normalverbraucher vorliegt.

13. Verfahren nach einem der Ansprüche 7-12, wobei eine Rückkehr der Schuppenerkrankung von 100 % für Myeloperoxidase nach der Behandlung vorliegt.

14. Verfahren nach einem der vorstehenden Ansprüche, wobei eine Differenz von mindestens 5 % zwischen Schuppen und keinen Schuppen vorliegt.

## Revendications

1. Procédé non invasif de diagnostic d'un état pelliculaire chez un sujet comprenant :
la détection d'un taux du biomarqueur myéloperoxydase dans un échantillon de cellules épithéliales/échantillon de cellules de peau obtenu auprès du sujet ; et
le diagnostic du sujet comme ayant un stress oxydatif et/ou un dommage oxydatif en fonction du taux d'un biomarqueur détecté, dans lequel il y a un changement du taux de biomarqueur par comparaison avec un taux de référence de biomarqueur ; et ainsi le diagnostic d'un état pelliculaire.

2. Procédé selon l'une quelconque des revendications précédentes dans lequel la collecte d'un échantillon de peau est choisi dans le groupe constitué d'articles adhésifs, d'arrachages de poils, d'un lavage de peau et de mélanges de ceux-ci.

3. Procédé selon l'une quelconque des revendications précédentes dans lequel le taux d'un ou de plusieurs autres biomarqueurs est détecté, dans lequel les un ou plusieurs autres biomarqueurs est/sont choisis dans le groupe constitué de (±)-9-hydroxy-10E, de 12Z-acide octadécadiénoïque et de (±)-13-hydroxy-10E, de 12Z-acide octadécadiénoïque (HODE), d'hydroperoxyde de squalène, de protéine de choc thermique 27 (HSP27), d'une modification oxydative de protéines, d'une oxydation de l'ADN et de nucléotides hydroxylés, d'isoprostanes, d'aldéhydes α,β-insaturés, de produits de réaction d'alcénals α,β-insaturés avec voie d'acide mercapturique et protéique, d'adduits de glycation précoces (EGA) et de produits terminaux de glycation avancés (AGE), d'antioxydants comme biomarqueurs de stress oxydatif et de mélanges de ceux-ci.

4. Procédé non invasif selon l'une quelconque des revendications précédentes, dans lequel le procédé comprend :
a) l'application d'un article adhésif sur un épithélium d'un mammifère ;
b) le fait de permettre l'adhérence des cellules épithéliales à l'article adhésif ;
c) le retrait de l'article adhésif de l'épithélium du mammifère ;
d) la préparation de l'article adhésif en utilisant des procédés standards de laboratoire pour l'extraction ;
e) l'extraction du biomarqueur myéloperoxydase des cellules épithéliales mises en adhésion sur ledit article adhésif ;
f) la mesure des biomarqueurs des cellules épithéliales mises en adhésion sur ledit article adhésif ;
g) la détermination de la quantité du biomarqueur dans les cellules épithéliales par comparaison avec un échantillon de référence après un traitement ; et
h) le diagnostic du sujet comme ayant un stress oxydatif et/ou un dommage oxydatif en fonction du taux d'un biomarqueur détecté, dans lequel il y a un changement de taux de biomarqueur par comparaison avec un taux de référence de biomarqueur ; et ainsi le diagnostic d'un état pelliculaire.

5. Procédé selon l'une quelconque des revendications précédentes, dans lequel le taux du biomarqueur détecté est normalisé en divisant le biomarqueur par une quantité de protéine sur l'article adhésif.

6. Procédé selon l'une quelconque des revendications précédentes dans lequel l'épithélium comprend le stratum corneum.

7. Procédé selon l'une quelconque des revendications précédentes dans lequel il y a un changement par rapport aux valeurs initiales dans le biomarqueur normalisé à la suite d'une application avec un traitement capillaire antifongique, par comparaison avec un taux de référence de biomarqueur avant l'application.

8. Procédé selon l'une quelconque des revendications précédentes dans lequel il y a une réduction d'au moins 40 % du biomarqueur normalisé sur une période de 3 semaines à la suite d'une application d'un shampooing anti-pelliculaire par comparaison avec un taux de référence de biomarqueur avant l'application.

9. Procédé selon l'une quelconque des revendications précédentes dans lequel il y a une réduction de 87 % du biomarqueur normalisé myéloperoxydase sur une période de 3 semaines à la suite d'une application d'un shampooing anti-pelliculaire par comparaison avec un taux de référence de biomarqueur myéloperoxydase avant l'application.

10. Procédé selon l'une quelconque des revendications précédentes dans lequel il y a un changement dans le biomarqueur normalisé à la suite d'une application d'un shampooing à la pyrithione de zinc par comparaison avec un taux de référence de biomarqueur avant l'application.

11. Procédé selon l'une quelconque des revendications précédentes dans lequel il y a un changement dans le biomarqueur normalisé à la suite d'une application d'un shampooing au sulfure de sélénium par comparaison avec un taux de référence de biomarqueur avant l'application.

12. Procédé selon l'une quelconque des revendications 7 à 11 dans lequel il y a une amélioration de l'état pelliculaire par comparaison avec une population normale.

13. Procédé selon l'une des revendications 7 à 12, dans lequel il y a un retour à 100 % de l'état pelliculaire pour la myéloperoxydase après traitement.

14. Procédé selon l'une quelconque des revendications précédentes dans lequel il y a une différence d'au moins 5 % entre un état pelliculaire et un état non-pelliculaire.
